# EUROPEAN PATENT SPECIFICATION

(11) **EP 0 434 202 B1**
(45) Date of publication and mention of the grant of the patent: **08.06.1994**
(21) Application number: 90312218.2
(22) Date of filing: 08.11.1990
(51) Int. Cl.: C07D 453/02, A61K 31/435

(54) **Azabicyclo amides and esters as 5-HT3 receptor antagonists**
Azabicycloamide und -ester als 5-HT3-Receptor-Antagonisten
Amides et esters azabicycliques comme antagonistes des récepteurs 5-HT3

(30) Priority: 17.11.1989 WO PCT/US89/05097
(43) Date of publication of application: 26.06.1991
(73) Proprietor: PFIZER INC., New York, N.Y. 10017 (US)
(72) Inventor: Rosen, Terry Jay, East Lyme, Connecticut (US)
(74) Representative: Bradbrook, Geoffrey William

(56) References cited:
- EP-A- 0 102 283
- EP-A- 0 221 702
- EP-A- 0 311 724
- US-A- 4 826 838

## Description

This invention relates to novel azabicyclo amides and esters which are antagonists at the serotonin 5-HT₃ receptor and useful as anti-emetic agents in warm blooded animals, particularly the emesis associated with the anticancer drug cisplatin. The 5-HT₃ receptor antagonists of the present invention are also useful in the treatment of schizophrenia, migraine, anxiety, cognitive disorders, Alzheimer's disease, pain and gastrointestinal disorders, such as irritable bowel syndrome.

Compounds recognized for their ability to act as antagonists at the serotonin 5-HT₃ receptor sites are described in U.S. Patents 4,593,034 and 4,749,718 and U.K. Patent Applications 2,125,398A, 2,166,726A, 2,166,727A, 2,166,728A and 2,193,633A. EP-A-0 311 724 discloses R-N-(1-azabicyclo[2.2.2]oct-3-yl)-benzamides which exhibit an anxiolytic activity.

The novel amides and esters of the present invention are of formula I and II: 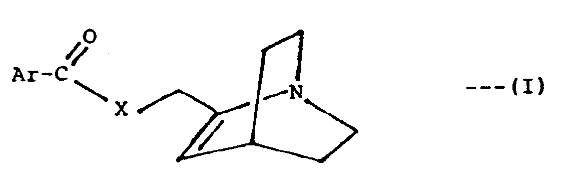
and 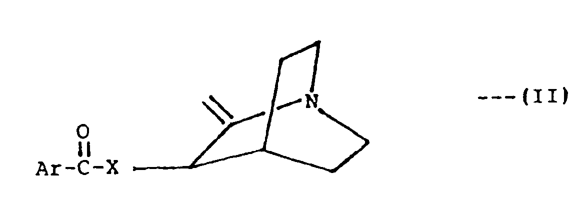
and a pharmaceutically acceptable acid addition salt, wherein Ar is an aromatic group such as phenyl, naphthyl, 3-indolyl, 3-indazolyl, 1-methyl-3-indolyl, 2-methoxyphenyl or 2-methoxy-4-amino-5-chlorophenyl; and X is O or NH.

Preferred are the compounds of formula I, where X is NH and Ar is 3-indolyl, 30-indazolyl or 2-methoxy-4-amino-5-chlorophenyl.

A second preferred group of compounds are those of formula II wherein X is O and Ar is 3-indolyl or 1-methyl-3-indolyl.

Also considered part of the present invention are the useful intermediates of the formula 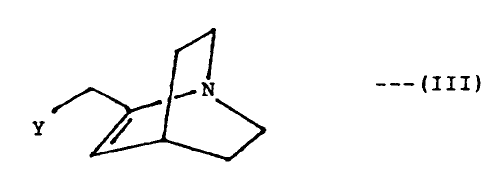
wherein Y is Cl, N₃, OH or NH₂.

The present invention also includes a method for treating emesis in a human being by administration of an anti-emetic amount of the compounds of formulae I and II and a pharmaceutical composition for said method comprising an effective amount of compounds of formulae I and II.

As previously indicated, the present invention embraces pharmaceutically acceptable salts of the biologically active compounds. Such salts are those which are non-toxic at the dosages administered. Since compounds of the invention contain basic groups, acid addition salts are possible. Pharmaceutically acceptable acid addition salts include e.g., the hydrochloride, hydrobromide, hydroiodide, sulfate, bisulfate, phosphate, acid phosphate, acetate, lactate, maleate, mesylate, fumarate, citrate, acid citrate, tartrate, bitartrate, succinate, gluconate, glutamate, aspartate and saccharate salts.

The compounds of formulae I and II wherein X is NH are prepared by acylation of the requisite amine with the appropriate acid as some reactive derivative. Such derivatives include acid halides, acid azides, acid cyanides, mixed acid anhydrides, active esters or active amides. Particularly preferred are acid halides, such as acid chlorides and active amides, such as acylimidazoles.

Acid chlorides are prepared by methods known to those skilled in the art and usually consist of reacting the acid with a chlorinating agent such as phosgene, thionyl chloride, phosphorous trichloride, phosphorus oxychloride, phosphorus pentachloride or oxalylchloride.

Acyl imidazole are readily prepared by reacting the appropriate acid with carbonyldiimidazole. The acyl imidazole can be generated in situ and used directly in the reaction, or it can be isolated prior to its use in the acylation reaction.

The acylation of the amine reagent is usually carried out in a reaction-inert solvent which is miscible with water. Such solvents include acetone, dimethylformamide, tetrahydrofuran, dimethylsulfoxide and dioxane.

In practice, equimolar amounts of the amine and acylating agent are combined in the appropriate solvent. Lesser or greater molar amounts of either reagents can be employed without changing the course of the reaction. When an acid chloride is employed as the acylating agent, it is preferred that a corresponding molar amount of acid scavenger be employed. Such scavengers include pyridine, triethylamine, etc.

Reaction temperature is not critical, and the acylation can readily be conducted at room temperature. At such a preferred reaction temperature, the reaction is substantially complete is about one to twelve hours.

The product is isolated by adding the water-miscible solvent to water or a salt solution thereof followed by extraction of the product with a water immiscible solvent such as methylene chloride or chloroform. On isolation, the product can be purified by classical methods such as recrystallization or column chromatography.

The compounds of formulae I and II wherein X is O are prepared by acylation of the appropriate alcohol with a reactive derivative of the requisite acid. Such derivatives are the same as those employed in the acylation of the amines as previously described. The preferred derivative is the acid chloride.

In practice, the acid chloride is added to a solution of an equimolar amount of the appropriate alcohol in a water soluble aprotic, reaction-inert solvent such as tetrahydrofuran. To facilitate the reaction it is preferred that an alkali metal salt of the alcohol be employed. This can readily be prepared by treating a solution of the alcohol with sodium hydride or an alkyl lithium such as butyl lithium, prior to the addition of the acid chloride.

Reaction temperature is not critical, and the acylation can readily be carried out at room temperature. At such a preferred reaction temperature, the reaction is complete in five to seven hours.

The product is obtained and purified by the same procedure as previously described for the amide products of the present invention.

The starting alcohol leading to the compounds of formula II (X=O) is prepared by the sodium borohydride reduction of the corresponding commercially available ketone.

Treatment of the resulting alcohol with thionyl chloride leads to an unexpected rearrangement and formation of the compound of formula III (Y=Cl). Treatment of III (Y=Cl) with a tetra alkyl ammonium azide at low temperatures leads to the synthesis of III (Y=N₃). Reduction of this azide with lithium aluminum hydride provides the intermediate III (Y=NH₂).

Treatment of the mesylate of the alcohol required to form compounds of formula II (X=O) with a tetra alkyl ammonium azide gives rise to a mixture of two azides as shown:

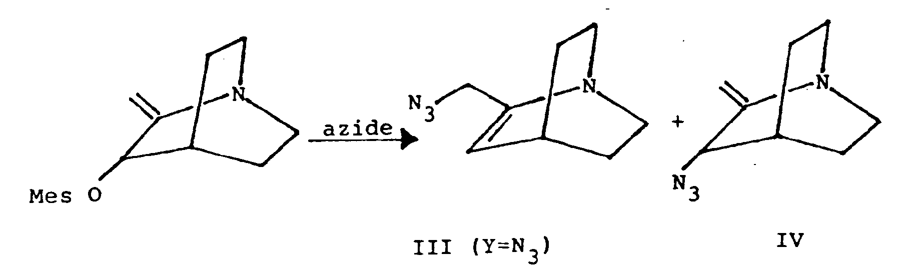

This mixture, which is comprised mainly of 2-methylene-3-azidoquinuclidine (Ca 2:1), can be reduced to a mixture of the corresponding amines. This mixture of amines can be used in the acylations previously described and the products subsequently separated and purified, or the mixture of amines can be converted to the respective t-butoxycarbonyl derivatives and separated. The separated t-butoxycarbonyl derivative can then be treated with dioxane saturated with hydrogen chloride resulting in the deblocking of the amine and isolation of the amine hydrochloride.

As previously mentioned, the compounds of the instant invention are antagonists of 5-hydroxytryptamine (5-HT) at the 5-HT₃ receptors. This property is demonstrated by their ability to antagonize the effects of 5-HT in the Bezold-Jarisch reflex [Richardson, et al., Nature 316, 126 (1985)] and their ability to bind to 5-HT receptors in brain tissue [Watling, et al., European J. Pharmacol. 149, 397 (1988)]. The compounds of the present invention are especially useful in controlling emesis due to administration of platinum anti-cancer agents. Evaluation of these compounds as anti-emetic agents against cisplastin uses the procedure in Cylys, Res. Commun. Chem. Pathol. Pharmacol., 23, 61 (1979).

The compounds of the present invention can be administered as antiemetic agents by either the oral or parenteral routes of administration, with the former being preferred for reasons of patient convenience and comfort. In general, these antiemetic compounds are normally administered orally in dosages ranging from 5 mg to 10 mg per kg of body weight per day and 0.1 mg to 1.0 mg per kg of body weight per day when given parenterally; variations will necessarily occur depending upon the condition of the subject being treated and the particular compound being administered. Typically, treatment is commenced at a low daily dosage and increased by the physician only if necessary. It is to be noted that these compounds may be administered in combination with pharmaceutically acceptable carriers by either of the routes previously indicated, and that such administration can be carried out in both single and multiple dosages.

The novel compounds of the invention can be orally administered in a wide variety of different dosage forms, i.e., they may be formulated with various pharmaceutically acceptable inert carriers in the form of tablets, capsules, lozenges, troches, hard candies, powders, sprays, aqueous suspensions, elixirs, syrups, and the like. Such carriers include solid diluents or fillers, sterile aqueous media and various non-toxic organic solvents, etc. Moreover, such oral pharmaceutical formulations can be suitably sweetened and/or flavored by means of various agents of the type commonly employed for such purposes. In general, the compounds of this invention are present in such oral dosage forms at concentration levels ranging from about 0.5% to about 90% by weight of the total composition, in amounts which are sufficient to provide the desired unit dosages.

For purposes of oral administration, tablets containing various excipients such as sodium citrate, calcium carbonate and calcium phosphate may be employed along with various disintegrants such as starch and preferably potato or tapioca starch, alginic acid and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred materials in this connection would also include lactose or milk sugar as well as high molecular weight polyethylene glycols. When aqueous suspensions and/or elixirs are desired of oral administration, the essential active ingredient therein may be combined with various sweetening or flavoring agents, coloring matter or dyes and, if so desired, emulsifying and/or suspending agents as well, together with such diluents as water, ethanol, propylene glycol, glycerin and various like combinations thereof.

The following examples illustrate the invention but are not to be construed as limiting the same.

### EXAMPLE 1

### N-(2,3-Dehydroquinuclidin-2-ylmethyl)-2-methoxy-4-amino-5-chlorobenzamide (I: Ar=2-CH₃O-4-NH₂-5-Cl-C₆H₂; X=NH)

Under a nitrogen atmosphere, in a round-bottom flask were placed 363 mg (1.8 mmol) of 2-methoxy-4-amino-5-chlorobenzoic acid and two mL of tetrahydrofuran. To the system was added 586 mg (3.6 mmol) of carbonyl diimidazole. The reaction mixture was stirred for 40 minutes, partitioned between chloroform and water and extracted with chloroform. The organic phase was dried (Na₂SO₄) and concentrated with a rotary evaporator. To the system was added 250 mg (1.8 mmol) of 2-aminomethyl-2,3-dehydroquinuclidine in two mL of tetrahydrofuran, and the reaction mixture was stirred at room temperature overnight. The reaction mixture was partitioned between chloroform and saturated aqueous sodium bicarbonate and extracted with chloroform. The organic phase was dried (Na₂SO₄) and concentrated, and the crude product was purified by flash column chromatography (25 g of silica gel) using 1:9 methanol/chloroform as the eluant to obtain 133 mg of white solid. This material was triturated with ether to obtain 85 mg of pure product as a white solid, mp 202-204°C. ¹HNMR (CDCl₃) delta 1.48 (m, 2H), 1.58 (m, 2H), 2.54 (m, 3H), 2.92 (m, 2H), 3.86 (s, 3H), 3.99 (d, 2H, J=6), 6.24 (s, 1H), 6.28 (d, 1H, J=6), 7.22 (s, 1H). HRMS: Calcd. for C₁₆H₂₀N₃O₂Cl³⁵: 321.1244. Found: 321.1198.

### EXAMPLE 2

Starting with the appropriate reagents and using the procedure of Example 1, the following compounds were prepared: 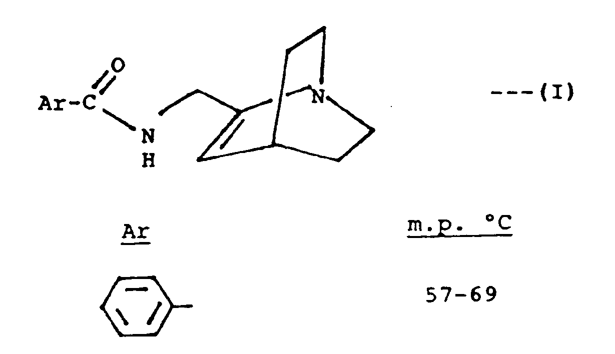 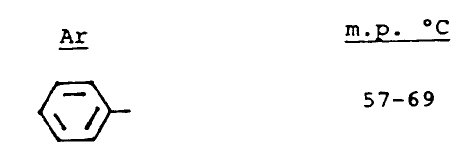
¹HNMR (CDCl₃) delta 1.34 (m,2H), 1.52 (m, 2H), 2.50 (m, 3H), 2.90 (m, 2H), 3.98 (d, 2H, J=6), 6.29 (d, 1H, J=6), 7.34 (m, 3H), 7.72 (d, 2H, J=6). HRMS: Calcd. for C₁₅H₁₈N₂O: 242. 1419. Found: 242.1388. Calcd. for C₁₅H₁₈N₂O x ½ H₂O: C, 71.67, H, 7.61, N, 11.14. Found: C, 71.56, H, 7.70, N, 11.03. 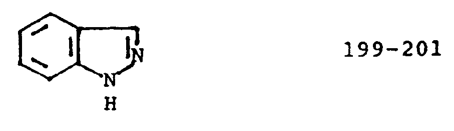
¹HNMR (CDCl₃) delta 1.64 (m, 4H), 2.74 (m, 3H), 3.12 (m, 2H), 4.08 (d, 2H, J=7), 6.68 (d, 1H, J=6), 7.20 (m, 1H), 7.34 (t, 1H, J=6), 7.42 (d, 1H, J=6), 8.42 (d, 1H, J=6). HRMS: Calcd. for C₁₆H₁₄N₄O: 282.1481. Found: 282.1487. 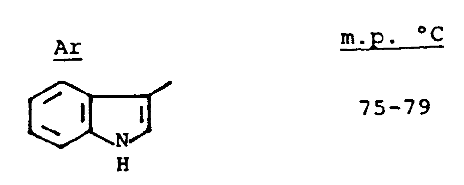
¹HNMR (CDCl₃) delta 1.38 (m, 2H), 1.54 (m, 2H), 2.54 (m, 3H), 2.82 (m, 2H), 4.06 (d, 2H, J=6), 6.36 (d, 1H, J=7), 7.18 (m, 2H), 7.38 (m, 1H), 7.66 (d, 1H, J=2), 7.96 (m, 1H). HRMS: Calcd. for C₁₇H₁₉N₃O: 281.1528. Found: 281.1552.

### EXAMPLE 3

### 1-Methylindole-3-(2-methylenequinuclidin-3-yl)carboxamide (II: Ar = 1-methyl-3-indolyl; and X = NH)

Under a nitrogen atmosphere, in a round-bottom flask were placed 0.36 mmol of a mixture of 2-amino-methyl-2,3-dehydroquinuclidine and 3-amino-2-methylenequinuclidine (ca 1:2) and 0.5 mL of tetrahydrofuran. To the system were added 105 mg (0.54 mmol) of 3-chlorocarbonyl-1-methylindole and 54 mg (0.075 mL, 0.54 mmol) of triethylamine. The reaction mixture was stirred at room temperature for ca. one hour, diluted with chloroform, washed with saturated aqueous sodium bicarbonate, dried (Na₂SO₄) and concentrated with a rotary evaporator. The crude material was subjected to flash column chromatography (10 g of silica gel) to obtain 30 mg of the desired product. This material was dissolved in ethyl acetate and hydrogen chloride was bubbled through the solution. The solution was concentrated with a rotary evaporator to obtain 22 mg of the corresponding hydrochloride as a tan solid, mp 160-167°C. ¹HNMR (DMSO-d₆) delta 1.84 (m, 1H), 2.02 (m, 2H), 2.26 (m, 2H), 3.25 (m, 3H), 3.88 (s, 3H), 5.06 (m, 1H), 5.46 (s, 1H), 5.94 (s, 1H), 7.23 (m, 2H), 7.54 (d, 1H, J=7), 8.16 (d, 1H, J=7), 8.26 (s, 1H), 8.37 (d, 1H, J=7). HRMS (free base): Calcd. for C₁₈H₂₁N₃O: 295.1684. Found: 295.1683.

### EXAMPLE 4

### N-(2-Methylenequinuclidin-3-yl)-2-methoxy-4-amino-5-chlorobenzamide (II: Ar = 2-CH₃O-4-NH₂-5-Cl-C₆H₂; and X = NH)

The titled amide product was prepared in a similar manner and has the following spectral properties: ¹HNMR (CDCl₃) delta 1.44 (m, 1H), 1.68 (m, 3H), 2.18 (m, 1H), 2.98 (m, 4H), 3.84 (s, 3H), 4.68 (d, 1H, J=8), 4.95 (d, 1H, J=2), 5.08 (d, 1H, J=2), 6.28 (s, 1H), 7.90 (d, 1H, J=8). HRMS: Calcd. for C₁₆H₂₀N₃O₂Cl³⁵: 321.1244. Found: 321.1235.

### EXAMPLE 5

### 2-Methylenequinuclidin-3-yl indole-3-carboxylate (II: Ar = 3-indolyl; and X = O)

Under a nitrogen atmosphere, in a round-bottom flask were placed 500 mg (3.1 mmol) of indole-3-carboxylic acid and three mL of tetrahydrofuran. To the system was added 0.30 mL (3.4 mmol) of oxalyl chloride over a period for five minutes, and the reaction mixture was stirred at room temperature for 1.5 hours and concentrated with a rotary evaporator. In a separate flask, under a nitrogen atmosphere, were placed 366 mg (2.63 mmol) of the alcohol 2-methylene-3-quinuclidinol and three mL of tetrahydrofuran. To this solution, cooled to 0°C, was added slowly 1.04 mL (2.6 mmol) of 2.5 M n-butyllithium in hexanes, and the mixture was stirred for five minutes and concentrated. The residue was dissolved in one mL of tetrahydrofuran and added to the acid chloride prepared above (two one mL rinses). The mixture was stirred at room temperature for five hours and partitioned between chloroform and saturated aqueous sodium bicarbonate. The layers were separated, and the aqueous phase was extracted with three portions of chloroform. The combined organic fractions were dried (Na₂SO₄) and concentrated. The crude material was purified by flash column chromatography (50 g of silica gel) using 1:19 methanol/chloroform as the eluant to obtain 435 mg of white foam which solidified, mp 99-109°C (dec.) ¹HNMR (CDCl₃) delta 1.54 (m, 1H), 1.76 (m, 2H), 2.04 (m, 1H), 2.34 (m, 1H), 3.10 (m, 5H), 5.17 (s, 1H), 5.32 (s, 1H), 5.68 (s, 1H), 7.26 (m, 1H), 7.41 (m, 1H), 7.84 (m, 1H), 8.14 (s, 1H). HRMS: Calcd. for C₁₇H₁₈N₂O₂:282.1369. Found: 282.1358.

### EXAMPLE 6

Employing the procedure of Example 5 and starting with the requisite reagents, the following compounds were prepared: 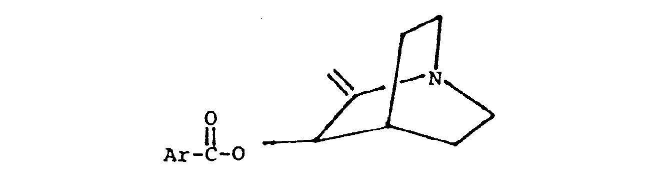 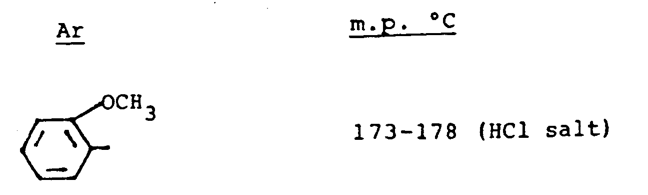
¹HNMR (DMSO-d₆) delta 2.0 (m, 4H), 3.44 (m, 5H), 3.86 (s, 3H), 5.62 (s, 1H), 5.76 (m, 1H), 5.88 (m, 1H), 7.05 (t, 1H, J=7), 7.19 (d, 1H, J=7), 7.60 (t, 1H, J=7), 7.74 (d, 1H, J=7). HRMS: Calcd. for C₁₆H₁₅NO₃: 273.1365. Found: 273.1340. 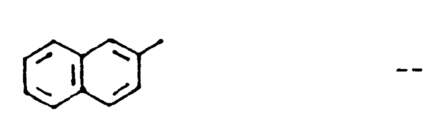
¹HNMR (CDCl₃) delta 2.02 (m, 2H), 2.16 (m, 1H), 2.40 (m, 1H), 2.71 (m, 1H), 3.48 (m, 2H), 3.64 (m, 2H), 5.70 (m, 1H), 5.86 (m, 1H), 6.48 (s, 1H), 7.62 (m, 2H), 7.94 (m, 4H), 8.58 (s, 1H). HRMS: Calcd. for C₁₉H₁₉NO₂: 293.1416. Found: 293.1431. 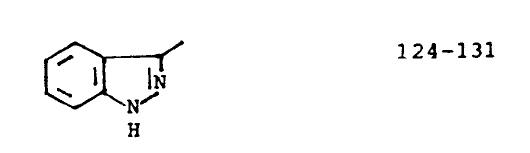
¹HNMR (CDCl₃) delta 1.58 (m, 1H), 1.82 (m, 2H), 2.06 (m, 1H), 2.42 (m, 1H), 3.12 (m, 4H), 5.22 (s, 1H), 5.37 (s, 1H), 5.85 (s, 1H), 7.30 (t, 1H, J=8), 7.44 (t, 1H, J=8), 7.78 (d, 1H, J=8) 8.14 (d, 1H, J=8). HRMS: Calcd. for C₁₆H₁₇N₃O₂: 283.1321. Found: 283.1302. 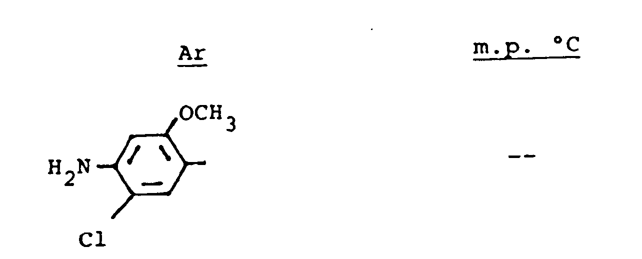
¹HNMR (CDCl₃) delta 1.48 (s, 1H), 1.70 (m, 2H), 1.94 (m, 1H), 2.24 (m, 1H), 3.02 (m, 4H), 3.82 (s, 3H), 5.04 (s, 1H), 5.28 (s, 1H), 5.53 (s, 1H), 7.23 (s, 1H), 7.76 (s, 1H). 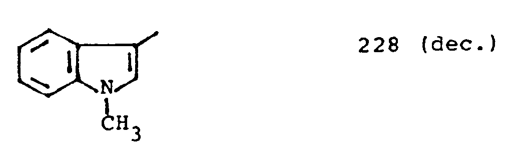
¹HNMR (DMSO-d₆) delta 2.00 (m, 3H), 2.24 (m, 1H), 3.46 (m, 5H), 3.92 (s, 3H), 5.65 (s, 1H), 5.84 (s, 1H), 5.98 (s, 1H), 7.31 (m, 2H), 7.61 (d, 1H, J=7), 8.00 (d, 1H, J=7), 8.30 (s, 1H). HRMS: Calcd. for C₁₈H₂₀N₂O₂: 296.1525. Found: 296.1504.

### EXAMPLE 7

### 2,3-Dehydroquinuclidin-2-ylmethyl indole-3-carboxylate (I: Ar = 3-indolyl; and X = O)

Under a nitrogen atmosphere, in a round-bottom flask are placed 20 mmol of potassium carbonate and 7 mL of water. To the system is added 10 mmol of the product of preparation B1 in 3 mL of tetrahydrofuran, and the mixture is stirred until thin layer chromatography indicates that no starting chloride remains. The mixture is diluted with water and extracted with several portions of methylene chloride. The combined dichloromethane fractions are dried (Na₂SO₄) and concentrated with a rotary evaporator, and the crude product is subjected to flash column chromatography to obtain 2-hydroxymethyl-2,3-dehydroquinuclidine (III, Y = OH). The procedure of Example 5 is repeated, replacing 2-methylene-3-quinuclidinol with III (Y = OH), to obtain the title compound.

### PREPARATION A

### 2-Methylene-3-Quinuclidinol

Under a nitrogen atmosphere, in a round-bottom flask were placed 16.7 g (122 mmol) of commercial 2-methylene-3-quinuclidinone and 250 mL of methanol. To this stirring solution was added 4.75 g (125 mmol) of sodium borohydride in portions over a period of 10 minutes. The reaction mixture was stirred at room temperature for 20 minutes, 200 mL of ethyl acetate was added to the system and the mixture was stirred for 10 minutes. To the system was added cautiously and slowly saturated aqueous sodium bicarbonate. To the mixture were added additional water and ethyl acetate, the layers were separated and the aqueous phase was extracted with ethyl acetate. The combined organic fractions were dried (Na₂SO₄) and concentrated. The crude material was purified by flash column chromatography (400 g of silica gel) using 1:9 methanol/chloroform as the eluant to obtain 3.8 g of product, mp 90-92°C. ¹HNMR (CDCl₃) delta 1.32 (m, 1H), 1.46 (m, 1H), 1.62 (m, 1H), 1.86 (m, 2H), 2.68 (m, 1H), 2.88 (m, 3H), 4.17 (d, 1H, J=2), 4.96 (d, 1H, J=2), 5.08 (d, 1H, J=2). HRMS: Calcd. for C₈H₁₃NO: 139.0998. Found: 139.0998. Calcd. for C₈H₁₃NO: C, 69.03, H, 9.41 N, 10.06. Found: C, 68.63, H, 9.24, N, 10.09.

### PREPARATION B

### 2-Aminomethyl-2,3-dehydroquinuclidine

### 1. 2-chloromethyl-2,3-dehydroquinuclidine

Under a nitrogen atmosphere, in a round-bottom flask were placed two g (14 mmol) of the product of Preparation A and 5 mL of methylene chloride, and the system was immersed in an ice bath. To the system was added 5.25 mL (72 mmol) of thionyl chloride dropwise over a period of 5 minutes. The ice bath was allowed to expire, and the reaction mixture was stirred at room temperature overnight. The reaction mixture was concentrated with a rotary evaporator, and 2N aqueous sodium hydroxide was added to the system. To the system was added water and the mixture was extracted with two portions of methylene chloride. The combined organic fractions were dried and concentrated to afford 1.9 g of the titled product as a yellow oil. ¹HNMR (CDCl₃) delta 1.38 (m, 2H), 1.56 (m, 2H), 2.56 (m, 3H), 2.94 (m, 2H), 3.96 (s, 2H), 6.48 (d, 1H, J=6). Calcd. for C₈H₁₂ClN x ½ H₂O: C, 57.65, H, 7.86, N, 8.40. Found: C, 57.92, H, 7.92, N, 8.17.

### 2. 2-azidomethyl-2,3-dehydroquinuclidine

Under a nitrogen atmosphere, in a round-bottom flask immersed in an ice/acetone bath were placed 3 g (19 mmol) of the product of Preparation B1 30 mL of acetonitrile, 2.65 mL (19 mmol) of triethylamine and 10 g (35 mmol) of tetra-n-butylammonium azide, and the reaction mixture was stirred for two hours, the temperature of the cold bath gradually rising to -5°C. The reaction mixture was poured into cold saturated aqueous sodium bicarbonate and extracted with cold ethyl acetate. The ethyl acetate solution was washed with three portions of cold aqueous sodium bicarbonate, dried (Na₂SO₄) and concentrated to obtain 8 g of crude product. This material was dissolved in cold ether/ethyl acetate and washed with five portions of cold aqueous sodium bicarbonate, dried (Na₂SO₄) and concentrated (rotary evaporator, cold water bath) to obtain 1.8 g of the allylic azide product as a yellow oil which was used immediately for the next transformation. ¹HNMR (CDCl₃) delta 1.32 (m, 2H), 1.50 (m, 2H), 2.28 (m, 2H), 2.94 (m, 2H), 3.26 (m, 1H), 3.65 (s, 2H), 6.37 (d, 1H, J=7). HRMS Calcd. for C₈H₁₂N₄: 164.1061. Found: 164.1025.

### 3. 2-Aminomethyl-2,3-dehydroquinuclidine

Under a nitrogen atmosphere, in a round-bottom flask were placed 22 mL (22 mmol) of 1M lithium aluminum hydride tetrahydrofuran, and the system was cooled in a dry ice/acetone bath. To the system was added dropwise over a period of ca. two minutes a solution of 1.8 g (11 mmol) of the compound of Preparation B2 in 7.8 mL of tetrahydrofuran and the cold bath was replaced with an ice/acetone bath. The reaction mixture was stirred for 30 minutes, the cold bath was removed and the mixture was stirred for an additional period of 30 minutes. The system was immersed in an ice/acetone bath, and 10 mL of 2N aqueous sodium hydroxide was added slowly and cautiously to the mixture. The system was removed from the cold bath, and the reaction mixture was stirred for 10 minutes. Sodium sulfate was added to the mixture, and after 15 minutes the solids were removed by suction filtration. The filtrate was concentrated with a rotary evaporator to obtain 1.67 g of product as a colorless oil which was used in subsequent transformations without further purification. ¹HNMR (CDCl₃) delta 1.34 (m, 2H), 1.50 (m, 2H), 2.48 (m, 3H), 2.90 (m, 2H), 3.21 (s, 2H), 6.17 (d, 1H, J=7). Mass spectrum, m/z 138 (parent).

### PREPARATION C

### 3-Amino-2-methylenequinuclidine Hydrochloride

### 1. 2-methylene-3-quinuclidinol mesylate

Under a nitrogen atmosphere, in a round-bottom flask were placed 8 g (58 mmol) of the alcohol of Preparation A and 60 mL of tetrahydrofuran. To the system (cooled in an ice/acetone bath) was added 17.9 mL (128 mmol) of triethylamine followed by 5.13 mL (66 mmol) of methanesulfonyl chloride over a period of 15 minutes. The mixture was gradually warmed to room temperature and stirred overnight. The reaction mixture was partitioned between 2N aqueous sodium hydroxide and chloroform, the layers were separated and the aqueous phase was extracted with chloroform. The combined chloroform fractions were dried (Na₂SO₄) and concentrated (rotary evaporator). The crude material was purified by flask column chromatography (300 g of silica gel) using 1:9 methanol/chloroform as the eluant to obtain 5.3 g of the desired product. ¹HNMR (CDCl₃) delta 1.48 (m, 2H), 1.68 (m, 1H), 1.84 (m, 1H), 2.28 (m, 1H), 2.76 (m, 1H), 2.94 (m, 3H), 3.04 (s, 3H), 5.08 (d, 1H, J=5), 5.14 (s, 1H), 5.24 (d, 1H, J=5).

### 2. Mixture of 2-methylene-3-azidoquinuclidine and 2-azidomethyl-2,3-dehydroquinuclidine

Under a nitrogen atmosphere were placed 5.3 g (24 mmol) of the product of Preparation Cl and 65 mL of acetonitrile. To the system was added 13.7 g (48 mmol) of tetra-n-butylammonium azide, and the reaction mixture was stirred at 55°C for 90 minutes and at room temperature overnight. The reaction mixture was partitioned between saturated aqueous sodium bicarbonate and chloroform, the layers were separated and the aqueous phase was extracted with chloroform. The combined chloroform fractions were dried (Na₂SO₄) and concentrated with a rotary evaporator. The crude material was purified by flash column chromatography (650 g of silica gel) using 1:9 methanol/chloroform as the eluant to obtain 5.5 g of colorless oil. This material was dissolved in chloroform and extracted with dilute aqueous hydrochloric acid. The aqueous extract was adjusted to a pH of ca. 7.5 and extracted with three portions of chloroform. The combined chloroform extracts were dried (Na₂SO₄) and concentrated to afford 3.46 of a mixture (ca. 2:1) of the titled azides, respectively.

### 3. Mixture of 2-methylene-3-aminoquinuclidine and 2-aminomethyl-2,3-dehydroquinuclidine

Under a nitrogen atmosphere, in a round-bottom flask were placed 42 mL (42 mmol) of 1M lithium aluminum hydride in tetrahydrofuran. The system was cooled to -78°C, and the azide mixture prepared above (3.46 g, 21.0 mmol) in 15 mL of tetrahydrofuran was added to the system dropwise. The mixture was stirred for 30 minutes, and the cold bath was replaced with an ice/acetone bath. The mixture was stirred for 30 minutes at room temperature, the cold bath was removed and the mixture was stirred at room temperature for one hour. The system was cooled in an ice/acetone bath and 20 mL of 2N aqueous sodium hydroxide was added cautiously and slowly to the system. To the system was added Na₂SO₄, the mixture was stirred for one hour, the solids were moved by suction filtration and filtrate was concentrated with a rotary evaporator to obtain 1.9 g of a mixture of the titled amines as a pale yellow oil. This mixture was used without further purification to obtain amides.

### 4. 2-methylene-3-aminoquinuclidine hydrochloride

Under a nitrogen atmosphere, in a round-bottom flask were placed 50 mg (0.36 mmol) of the mixture of amines from Preparation C2 and 0.5 mL of di-tert-butyldicarbonate, and the reaction mixture was stirred at room temperature for three days. The mixture was partitioned between dichloromethane and saturated aqueous sodium bicarbonate, and the layers were separated. The aqueous phase was extracted with dichloromethane, and the combined organic fractions were dried (Na₂SO₄) and concentrated with a rotary evaporator. The crude material was subjected to flash column chromatography (15 g of silica gel) to obtain 46 mg of the t-butoxycarbonyl derivative of 2-methylene-3-aminoquinuclidine ¹HNMR (CDCl₃) delta 1.46 (s, 9H), 1.68 (m, 3H), 2.09 (s, 1H), 2.92 (m, 4H), 4.26 (m, 1H), 4.76 (m, 1H), 4.95 (s, 1H), 5.09 (s, 1H). Mass spectrum, m/z 248 (parent) and 13 mg of the t-butoxycarbonyl derivative of 2-aminomethyl-2,3-dehydroquinuclidine. ¹HNMR (CDCl₃) delta 1.44 (m, 10H), 1.6 (m, 2H), 2.16 (m, 1H), 2.52 (m, 3H), 2.92 (m, 2H), 3.70 (m, 2H), 6.28 (d, 1H, J=7). Mass spectrum, m/z 248 (parent).

Under a nitrogen atmosphere, in a round-bottom flask were placed 29 mg (0.12 mmol) of the t-butoxycarbonyl derivatives of 2-methylene-3-aminoquinuclidine and 0.8 mL of dioxane saturated with hydrogen chloride. The mixture was stirred at room temperature for 90 minutes and concentrated with a rotary evaporator to obtain 34 mg of the hydrochloride salt of the product as a white solid. ¹HNMR (DMSO-d₆) delta 1.92 (m, 3H), 2.10 (m, 1H), 2.44 (m, 1H), 3.24 (m, 1H), 3.46 (m, 3H), 4.30 (m, 1H), 5.93 (s, 1H), 6.03 (s, 1H). Mass spectrum, m/z 238 (parent).

## Claims (Claims for the following Contracting State(s): DE, GB, FR, IT, NL, SE, LI, CH, BE, AT, LU, DK)

1. A compound of the formula 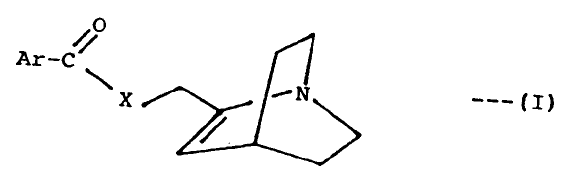 and 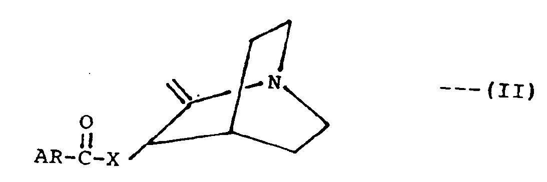 and a pharmaceutically acceptable acid addition salt thereof wherein Ar is phenyl, naphthyl, 3-indolyl, 3-indazolyl, 1-methyl-3-indolyl, 2-methoxyphenyl or 2-methoxy-4-amino-5-chlorophenyl; and X is O or NH.

2. A compound of claim 1, formula I, wherein X is NH.

3. The compound of claim 2, wherein Ar is 3-indolyl.

4. The compound of claim 2, wherein Ar is 3-indazolyl.

5. The compound of claim 2, wherein Ar is 2-methoxy-4-amino-5-chlorophenyl.

6. A compound of claim 1, formula II, wherein X is O.

7. The compound of claim 6, wherein AR is 3-indolyl.

8. The compound of claim 6, wherein Ar is 1-methyl-3-indolyl.

9. A compound of the formula 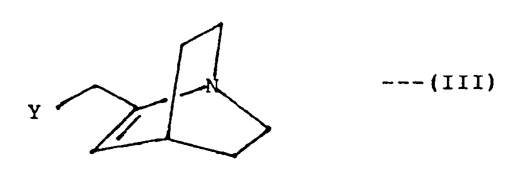 wherein Y is OH, Cl, N₃ or NH₂.

10. A pharmaceutical composition comprising an anti-emetic effective amount of a compound according to any one of claims 1 to 8 and a pharmaceutically acceptable diluent or carrier.

11. A compound according to any one of claims 1 to 8, for use in medicine.

12. Use of a compound according to any one of claims 1 to 8 for making a medicament for treating emesis.

## Claims (Claims for the following Contracting State(s): GR)

1. A process for preparing a compound of the formula 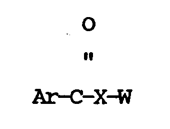 or a pharmaceutically acceptable salt thereof wherein Ar is phenyl, naphthyl, 3-indolyl, 3-indazolyl, 1-methyl-3-indolyl, 2-methoxyphenyl or 2-methoxy-4-amino-5-chlorophenyl; X is O or NH; and W is 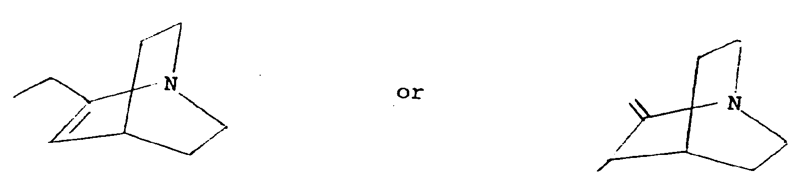 which comprises reacting a reactive derivative of an acid of the formula 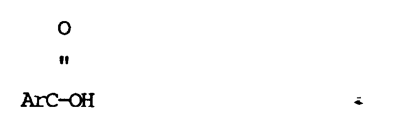 with a compound of the formula
H-X-W or M-X-W
wherein M is an alkali metal salt with the proviso that when M is an alkali metal salt, X is O, in a reaction inert solvent until the reaction is substantially complete.

2. A process according to claim 1, formula I, wherein X is NH.

3. A process according to claim 2, wherein Ar is 3-indolyl.

4. A process according to claim 2, wherein Ar is 3-indazolyl.

5. A process according to claim 2, wherein Ar is 2-methoxy-4-amino-5-chlorophenyl.

6. A process according to claim 1, formula II, wherein X is O.

7. A process according to claim 6, wherein Ar is 3-indolyl.

8. A process according to claim 6, wherein Ar is 1-methyl-3-indolyl.

9. An intermediate of the formula 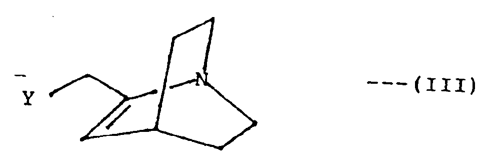 wherein Y is OH, Cl, N₃ or NH₂.

## Claims (Claims for the following Contracting State(s): ES)

1. A process for preparing a compound of the formula 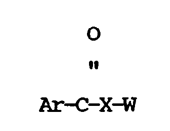 or a pharmaceutically acceptable salt thereof wherein Ar is phenyl, naphthyl, 3-indolyl, 3-indazolyl, 1-methyl-3-indolyl, 2-methoxyphenyl or 2-methoxy-4-amino-5-chlorophenyl; X is O or NH; and W is 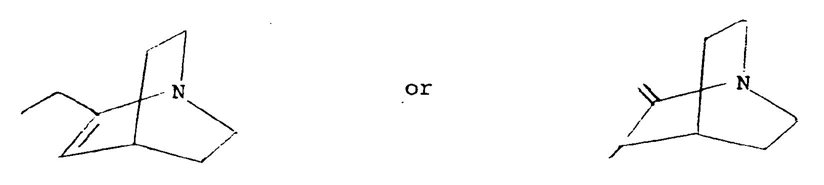 which comprises reacting a reactive derivative of an acid of the formula 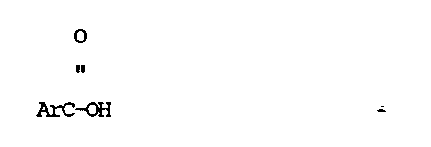 with a compound of the formula
H-X-W or M-X-W
wherein M is an alkali metal salt with the proviso that when M is an alkali metal salt, X is O, in a reaction inert solvent until the reaction is substantially complete.

2. A process according to claim 1, formula I, wherein X is NH.

3. A process according to claim 2, wherein Ar is 3-indolyl.

4. A process according to claim 2, wherein Ar is 3-indazolyl.

5. A process according to claim 2, wherein Ar is 2-methoxy-4-amino-5-chlorophenyl.

6. A process according to claim 1, formula II, wherein X is O.

7. A process according to claim 6, wherein Ar is 3-indolyl.

8. A process according to claim 6, wherein Ar is 1-methyl-3-indolyl.

## Patentansprüche (Patentansprüche für folgende(n) Vertragsstaat(en): DE, GB, FR, IT, NL, SE, LI, CH, BE, AT, LU, DK)

1. Verbindung der Formel 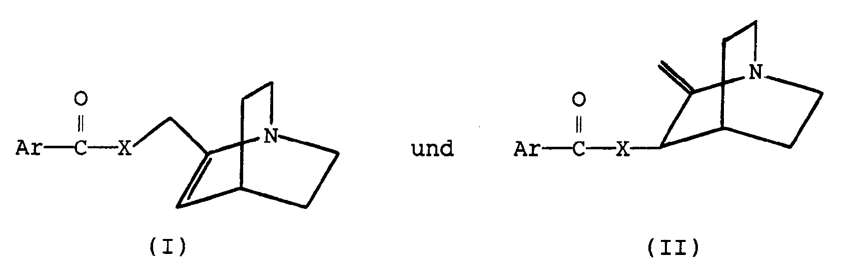 und ein pharmazeutisch verträgliches Säureadditionssalz derselben, worin Ar Phenyl, Naphthyl, 3-Indolyl, 3-Indazolyl, 1-Methyl-3-indolyl, 2-Methoxyphenyl oder 2-Methoxy-4-amino-5-chlorphenyl ist und X O oder NH bedeutet.

2. Verbindung nach Anspruch 1, Formel I, worin X NH ist.

3. Verbindung nach Anspruch 2, worin Ar 3-Indolyl ist.

4. Verbindung nach Anspruch 2, worin Ar 3-Indazolyl ist.

5. Verbindung nach Anspruch 2, worin Ar 2-Methoxy-4-amino-5-chlorphenyl ist..

6. Verbindung nach Anspruch 1, Formel II, worin X O ist.

7. Verbindung nach Anspruch 6, worin Ar 3-Indolyl ist.

8. Verbindung nach Anspruch 6, worin Ar 1-Methyl-3-indolyl ist.

9. Verbindung der Formel 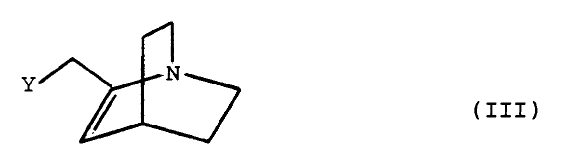 worin Y OH, Cl, N₃ oder NH₂ ist.

10. Pharmazeutische Zusammensetzung, enthaltend eine anti-emetisch wirksame Menge einer Verbindung nach einem der Ansprüche 1 bis 8 und ein pharmazeutisch verträgliches Verdünnungsmittel oder Träger.

11. Verbindung nach irgendeinem der Ansprüche 1 bis 8 für die Verwendung in der Medizin.

12. Die Verwendung einer Verbindung nach einem der Ansprüche 1 bis 8 zur Herstellung eines Medikaments zur Behandlung des Erbrechens.

## Patentansprüche (Patentansprüche für folgende(n) Vertragsstaat(en): GR)

1. Verfahren zur Herstellung einer Verbindung der Formel 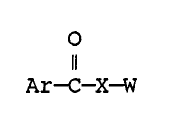 oder eines pharmazeutisch verträglichen Salzes derselben, worin Ar Phenyl, Naphthyl, 3-Indolyl, 3-Indazolyl, 1-Methyl-3-Indolyl, 2-Methoxyphenyl oder 2-Methoxy-4-amino-5-chlorphenyl ist und X O oder NH bedeutet, und W die nachfolgenden Formeln aufweist, 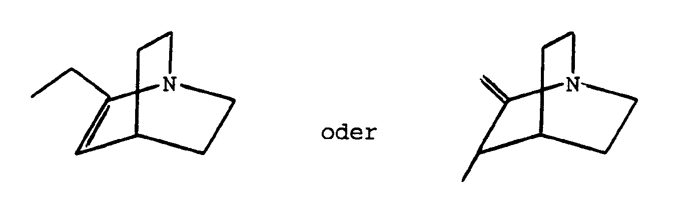 welches das Umsetzen eines reaktiven Derivats einer Säure der Formel 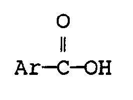 mit einer Verbindung der Formel
H-X-W oder M-X-W
umfaßt, worin M ein Alkalimetallsalz ist, unter der Bedingung, daß, falls M ein Alkalimetallsalz ist, X gleich O ist, in einem reaktions-inerten Lösungsmittel, bis die Reaktion im wesentlichen vollständig ist.

2. Verfahren nach Anspruch 1, Formel I, worin X NH bedeutet.

3. Verfahren nach Anspruch 2, worin Ar 3-Indolyl ist.

4. Verfahren nach Anspruch 2, worin Ar 3-Indazolyl ist.

5. Verfahren nach Anspruch 2, worin Ar 2-Methoxy-4-amino-5-chlorphenyl ist.

6. Verfahren nach Anspruch 1, Formel II, worin X O ist.

7. Verfahren nach Anspruch 6, worin Ar 3-Indolyl ist.

8. Verfahren nach Anspruch 6, worin Ar 1-Methyl-3-Indolyl ist.

9. Zwischenverbindung der nachstehenden Formel III 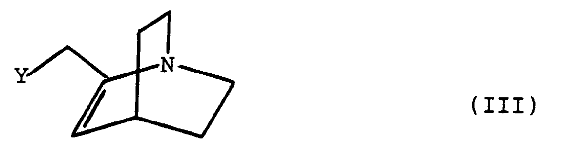 worin Y OH, Cl, N₃ oder NH₂ ist.

## Patentansprüche (Patentansprüche für folgende(n) Vertragsstaat(en): ES)

1. Verfahren zur Herstellung einer Verbindung der Formel 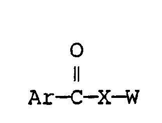 oder eines pharmazeutisch verträglichen Salzes derselben, worin Ar Phenyl, Naphthyl, 3-Indolyl, 3-Indazolyl, 1-Methyl-3-Indolyl, 2-Methoxyphenyl oder 2-Methoxy-4-amino-5-chlorphenyl ist und X O oder NH bedeutet, und W die nachfolgenden Formeln aufweist, 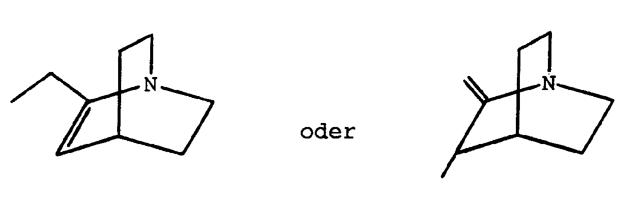 welches das Umsetzen eines reaktiven Derivats einer Säure der Formel 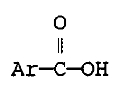 mit einer Verbindung der Formel
H-X-W oder M-X-W
umfaßt, worin M ein Alkalimetallsalz ist, unter der Bedingung, daß, falls M ein Alkalimetallsalz ist, X gleich O ist, in einem reaktions-inerten Lösungsmittel, bis die Reaktion im wesentlichen vollständig ist.

2. Verfahren nach Anspruch 1, Formel I, worin X NH bedeutet.

3. Verfahren nach Anspruch 2, worin Ar 3-Indolyl ist.

4. Verfahren nach Anspruch 2, worin Ar 3-Indazolyl ist.

5. Verfahren nach Anspruch 2, worin Ar 2-Methoxy-4-amino-5-chlorphenyl ist.

6. Verfahren nach Anspruch 1, Formel II, worin X O ist.

7. Verfahren nach Anspruch 6, worin Ar 3-Indolyl ist.

8. Verfahren nach Anspruch 6, worin Ar 1-Methyl-3-Indolyl ist.

## Revendications (Revendications pour l'(les) Etat(s) contractant(s) suivant(s): DE, GB, FR, IT, NL, SE, LI, CH, BE, AT, LU, DK)

1. Composé de formules 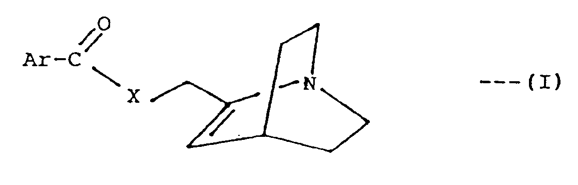 et 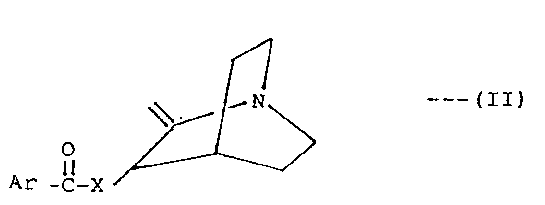 et un sel d'addition pharmaceutiquement acceptable de ce composé, formules dans lesquelles Ar est un groupe phényle, naphtyle, 3-indolyle, 3-indazolyle, 1-méthyl-3-indolyle, 2-méthoxyphényle ou 2-méthoxy-4-amino-5-chlorophényle ; et X représente O ou NH.

2. Composé suivant la revendication 1, de formule I dans laquelle X représente NH.

3. Composé suivant la revendication 2, dans lequel Ar est un groupe 3-indolyle.

4. Composé suivant la revendication 2, dans lequel Ar est un groupe 3-indazolyle.

5. Composé suivant la revendication 2, dans lequel Ar est un groupe 2-méthoxy-4-amino-5-chlorophényle.

6. Composé suivant la revendication 1, de formule II dans laquelle X représente O.

7. Composé suivant la revendication 6, dans lequel Ar est un groupe 3-indolyle.

8. Composé suivant la revendication 6, dans lequel Ar est un groupe 1-méthyl-3-indolyle.

9. Composé de formule 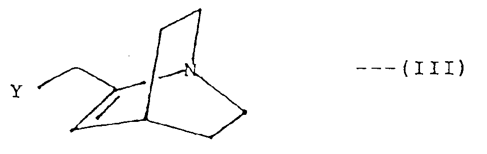 dans laquelle Y représente OH, Cl, N₃ ou NH₂.

10. Composition pharmaceutique comprenant une quantité à effet anti-émétique d'un composé suivant l'une quelconque des revendications 1 à 8 et un diluant ou support acceptable du point de vue pharmaceutique.

11. Composé suivant l'une quelconque des revendications 1 à 8, destiné à être utilisé en médecine.

12. Utilisation d'un composé suivant l'une quelconque des revendications 1 à 8 pour la préparation d'un médicament destiné au traitement du vomissement.

## Revendications (Revendications pour l'(les) Etat(s) contractant(s) suivant(s): GR)

1. Procédé de production d'un composé de formule 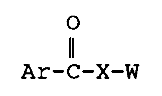 ou d'un sel acceptable du point de vue pharmaceutique de ce composé, dans lequel Ar est un groupe phényle, naphtyle, 3-indolyle, 3-indazolyle, 1-méthyl-3-indolyle, 2-méthoxyphényle ou 2-méthoxy-4-amino-5-chlorophényle ; et X représente O ou NH ; et W représente 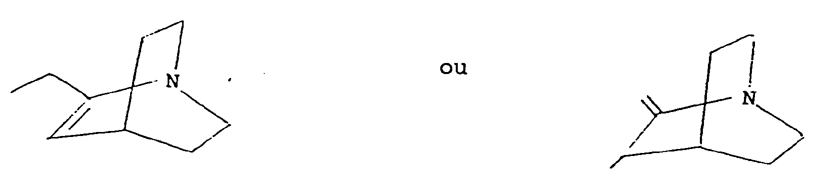 qui comprend la réaction d'un dérivé réactif d'un acide de formule 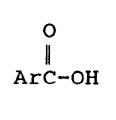 avec un composé de formule
H-X-W ou M-X-W
dans laquelle M est un sel de métal alcalin, sous réserve que lorsque M est un sel de métal alcalin, X représente O, dans un solvant inerte vis-à-vis de la réaction jusqu'à ce que cette dernière soit principalement terminée.

2. Procédé suivant la revendication 1, dans lequel X représente NH dans la formule I.

3. Procédé suivant la revendication 2, dans lequel Ar est un groupe 3-indolyle.

4. Procédé suivant la revendication 2, dans lequel Ar est un groupe 3-indazolyle.

5. Procédé suivant la revendication 2, dans lequel Ar est un groupe 2-méthoxy-4-amino-5-chlorophényle.

6. Procédé suivant la revendication 1, dans lequel X représente O dans la formule II.

7. Procédé suivant la revendication 6, dans lequel Ar est un groupe 3-indolyle.

8. Procédé suivant la revendication 6, dans lequel Ar est un groupe 1-méthyl-3-indolyle.

9. Composé intermédiaire de formule 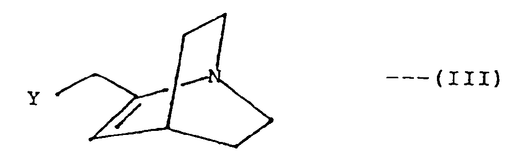 dans laquelle Y représente OH, Cl, N₃ ou NH₂.

## Revendications (Revendications pour l'(les) Etat(s) contractant(s) suivant(s): ES)

1. Procédé de production d'un composé de formule 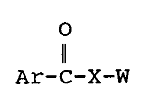 ou d'un sel acceptable du point de vue pharmaceutique de ce composé, dans lequel Ar est un groupe phényle, naphtyle, 3-indolyle, 3-indazolyle, 1-méthyl-3-indolyle, 2-méthoxyphényle ou 2-méthoxy-4-amino-5-chlorophényle ; et X représente O ou NH ; et W représente 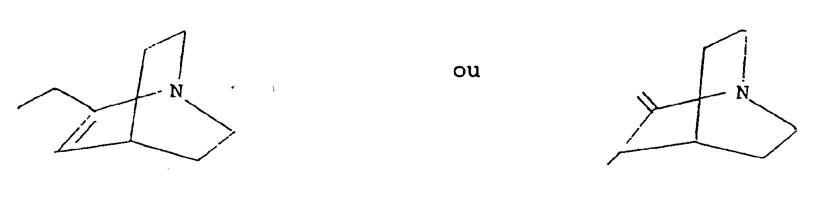 qui comprend la réaction d'un dérivé réactif d'un acide de formule 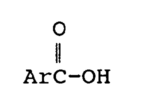 avec un composé de formule
H-X-W ou M-X-W
dans laquelle M est un sel de métal alcalin, sous réserve que lorsque M est un sel de métal alcalin, X représente O, dansun solvant inerte vis-à-vis de la réaction jusqu'à ce que cette dernière soit principalement terminée.

2. Procédé suivant la revendication 1, dans lequel X représente NH dans la formule I.

3. Procédé suivant la revendication 2, dans lequel Ar est un groupe 3-indolyle.

4. Procédé suivant la revendication 2, dans lequel Ar est un groupe 3-indazolyle.

5. Procédé suivant la revendication 2, dans lequel Ar est un groupe 2-méthoxy-4-amino-5-chlorophényle.

6. Procédé suivant la revendication 1, dans lequel X représente O dans la formule II.

7. Procédé suivant la revendication 6, dans lequel Ar est un groupe 3-indolyle.

8. Procédé suivant la revendication 6, dans lequel Ar est un groupe 1-méthyl-3-indolyle.
